# EUROPEAN PATENT SPECIFICATION

(11) **EP 1 782 766 B1**
(45) Date of publication and mention of the grant of the patent: **23.02.2011**
(21) Application number: 06255671.7
(22) Date of filing: 03.11.2006
(51) Int. Cl.: A61F 2/90

(54) **Stent with strain concentrating bridge**
Mit einer Dehnungs-Konzentrationsbrücke versehener Stent
Stent à pont de concentration de contraintes

(30) Priority: 03.11.2005 US 266535
(43) Date of publication of application: 09.05.2007
(73) Proprietor: Nitinol Development Corporation, Fremont, California 94539 (US)
(72) Inventor: Bonsignore, Craig, Pleasanton, CA 94566 (US); Carlson, John E., Morrow, OH 45152 (US); Shaw, William, Cincinnati, OH 45249 (US)
(74) Representative: Belcher, Simon James

(56) References cited:
- EP-A- 1 516 600
- WO-A-00/62708
- US-A1- 2005 182 479

## Description

The invention relates to intraluminal medical devices. More particularly, the invention relates to a stent having at least one strain concentrating bridge that releasably connects adjacent segments of a stent when subjected to loading conditions exceeding a specified threshold.

Percutaneous transluminal angioplasty (PTA) is a therapeutic medical procedure used to increase blood flow through an artery. In this procedure, an angioplasty balloon is inflated within the stenosed vessel, or body passageway, in order to shear and disrupt the wall components of the vessel to obtain an enlarged lumen. A dissection "flap" of underlying tissue can occur, however, which can undesirably fold into and close off the lumen. Immediate corrective surgery becomes necessary as a result.

More recently, transluminal prosthesis, such as stents, have been used for implantation in blood vessels, biliary ducts, or other similar organs of a patient in order to open, dilate or maintain the patency thereof. An example of such a stent is given in US-4,733,665 to Palmaz. Such stents are often referred to as balloon expandable stents. A balloon expandable stent is typically made from a solid tube of stainless steel having a series of cuts made therein. The stent has a first smaller diameter, permitting the stent to be crimped onto a balloon catheter for delivery through the human vasculature to an intended treatment site. The stent also has a second, expanded diameter, that is achieved by the application of a radially, outward directed force by the balloon catheter from the interior of the tubular shaped stent when located at the intended treatment site.

Such balloon stents are often impractical for use in some vessels, such as the carotid artery. The carotid artery is easily accessible and close to the surface of a patient's skin. Thus, emplacement of a balloon expandable stent in such a vessel poses severy injury risks to a patient through even day-to-day activities, particularly where a force to the patient's neck could result in collapse of the stent within the vessel. Self-exapnding stents have thus been devised in part to address these risks, wherein the self-expanding stent will recover its expanded state after being temporarily crushed by a force applied to a patient's neck or the like.

One type of self-expanding stent is disclosed in US-4,655,771. The stent disclosed in US-4,655,771 has a radially and axially flexible, elastic tubular body with a pre-determined diameter that is variable under axial movement of the ends of the body relative to each other and which is composed of a plurality of individually rigid but flexible and elastic thread elements defining a radially self-expanding helix. This type of stent is known in the art as a "braided stent" and is so designated herein. Placement of such braided stents in a body vessel can be achieved by a device which comprises an outer catheter for holding the stent at its distal end, and an inner piston which pushes the stent forward once it is in position.

Braided stents have many disadvantages, however, including insufficient radial strength to effectively hold open a diseased vessel. In addition, the plurality of wires or fibers comprising a braided stent become dangerous if separated from the body of the stent as they could pierce through the vessel. Tube-cut stents made from alloys having shape memory and/or superelastic characteristics have thus been developed to address some of the concerns posed by braided stents.

The shape memory characteristics allow the devices to be deformed to facilitate insertion into a body lumen or cavity, whereafter resumption of the original form of the stent occurs when subjected to sufficient heat from the patient's body, for example. Superelastic characteristics, on the other hand, generally allow the stent to be deformed and restrained in the deformed condition to facilitate insertion of the stent into the patient's body, wherein the deformation of the stent causes a phase transformation in the materials comprising the stent. Once within the body lumen of the patient, the restraint on the superelastic stent is removed and the superelastic stent returns to its original un-deformed state.

Alloys having shape memory/superelastic characteristics generally have at least two phases. These phases are a martensite phase, which has a relatively low tensile strength and which is stable at relatively low temperatures, and an austentite phase, which has a relatively high tensile strength and which is stable at temperatures higher than the martensite phase.

Shape memory characteristics are imparted to an alloy by heating the alloy to a temperature above which the transformation from the martensite phase to the austenite phase is complete, i.e., a temperature above which the austenite phase is stable (the A_{f} temperature). The shape of the metal during this heat treatment is the shape "remembered". The heat-treated alloy is cooled to a temperature at which the martensite phase is stable, causing the austenite phase to transform to the martensite phase. The alloy in the martensite phase is then plastically deformed, e.g., to facilitate the entry thereof into a patient's body. Subsequent heating of the deformed martensite phase to a temperature above the martensite to austenite transformation temperature causes the deformed martensite phase to transform to the austenite phase, and during this phase transformation the alloy reverts back to its original shape if unrestrained. If restrained, the metal will remain martensitic until the restraint is removed.

Methods of using the shape memory characteristics of these alloys in medical devices intended to be placed within a patient's body present operational difficulties. For example, with shape memory alloys having a stable martensite temperature below body temperature, it is frequently difficult to maintain the temperature of the medical device containing such an alloy sufficiently below body temperature to prevent the transformation of the martensite phase to the austenite phase when the device was being inserted into a patient's body. With intravascular devices formed of shape memory alloys having martensite-to-austenite transformation temperatures well above body temperature, the devices can be introduced into a patient's body with little or no problem, but they must be heated to the martensite-to-austenite transformation temperature which is frequently high enough to cause tissue damage.

When stress is applied to a specimen of an alloy or metal such as Nitinol exhibiting superelastic characteristics at a temperature above which the austenite is stable (i.e., the temperature at which the transformation of martensite phase to the austenite phase is complete), the specimen deforms elastically until it reaches a particular stress level where the alloy then undergoes a stress-induced phase transformation from the austenite phase to the martensite phase. As the phase transformation proceeds, the alloy undergoes significant increases in strain, but with little or no corresponding increases in stress. The strain increases while the stress remains essentially constant until the transformation of the austenite phase to the martensite phase is complete. Thereafter, further increases in stress are necessary to cause further deformation. The martensitic alloy or metal first deforms elastically upon the application of additional stress and then plastically with permanent residual deformation.

If the load on the specimen is removed before any permanent deformation has occurred, the martensitic specimen will elastically recover and transform back to the austenite phase. The reduction in stress first causes a decrease in strain. As stress reduction reaches the level at which the martensite phase transforms back into the austenite phase, the stress level in the specimen will remain essentially constant (but substantially less than the constant stress level at which the austenite transforms to the martensite) until the transformation back to the austenite phase is complete, i.e., there is significant recovery in strain with only negligible corresponding stress reduction. After the transformation back to the austenite phase is complete, further stress reduction results in elastic strain reduction. This ability to incur significant strain at relatively constant stress upon the application of a load, and to recover from the deformation upon the removal of the load, is commonly referred to as superelasticity or pseudoelasticity. It is this property of the material which makes it useful in manufacturing tube cut self-expanding stents.

The compressive forces associated with stent loading and deployment can pose concerns with respect to self-expanding stents. In stent designs having periodically positioned bridges, for example, the resulting gaps between unconnected loops may be disadvantageous. In both the loading and the deployment thereof, the stent is constrained to a small diameter and subjected to high compressive axial forces. These forces are transmitted axially through the stent by the connecting bridges and may cause undesirable buckling or compression of the adjacent loops in the areas where the loops are not connected by bridges.

Other concerns with self-expanding stents include reduced radiopacity, often resulting in the attachment of markers to the stent. The attached markers tend to increase the profile of the stent, and can dislodge from the stent or otherwise compromise the performance of the stent.

A still further concern is the transmission of forces between interconnected elements of a stent. Conventional vascular stents tend to comprise a series of ring-like radially expandable structural members that are axially connected by bridging elements. When a stent is subjected to *in vivo* bending, stretching or compression, due to physiologic dynamics of the patient, its ring-like structural members distribute themselves accordingly to conform the structural members of the stent to its vascular surroundings. These loading conditions cause the ring-like structural members to change their axial positions relative to one another. The bridging elements help to constrain the ring-like structural members and therefore propagate strain between the ring-like structural members. The axial and radial expansion of the otherwise constrained stent, and the bending of the stent, that occurs during delivery and deployment, often renders conventional interconnected stents susceptible to fatigue fractures. Physiologic dynamics within the body of a patient also contribute to fatigue fractures of conventional stents.

Historically, therefore, stents have been designed to remain contiguous within the body. However, there may be instances where it may be desirable to have a stent which is separable within the body, such as in blood vessels subjected to longitudinal elongation or excessive compression or bending. In such cases, a frangible stent may prove useful to achieve good vessel opposition or to minimize displacement of the expanded stent into the lumen area of a vessel. The cyclic strains, due to physiologic dynamics or otherwise, that can propagate through and cause damage to the structures of a stent can be minimized where portions of the stent physically separate within the body.

Even where connected strut segments have been designed to disconnect upon deployment in order to minimize the occurrence of fatigue fractures, such as in co-pending U.S. Patent Application Serial No. 10/687,143, filed October 18, 2003, of common assignment herewith and published as US-A-2005/0085896 on 21 April 2005, such stents can prove unstable and susceptible to tipping or rotation within a vessel, particularly during delivery, particularly where the L/D ratio, i.e., the ratio of a expanded strut length L to an expanded diameter D of the stent, is greater than one. On the other hand, where the L/D ratio approaches zero, particularly where L approaches zero, then uniform and predictable positioning of the various segments comprising a stent is compromised as segments tend to de-couple before becoming firmly opposed to the lumen of the intended blood vessel. Unpredictable propelling of the segments from the delivery device can also occur.

In the commonly owned and co-pending U.S. Patent Application Serial No. 10/779,493, filed February 13, 2004 and published August 18, 2005 as US-A-2005/0182479, adjacent rings of an intraluminal stent device are connected by frangible bridge members comprised of polymeric materials. The polymeric bridge is weaker than the adjacent rings so that as a level of strain beyond a threshold level is experienced, the bridge yields before the adjacent rings yield. In practice, the stent is delivered with its adjacent rings connected, whereas after deployment the polymeric bridges may yield to separate one or more of adjacent rings from another adjacent ring when the bridges are subjected to sufficient strain. The polymeric bridge feature does not account for bridges comprised of other materials, such as metals, however, and does not address various dimensional or other alterations in the bridge or rings that could accommodate various strain threshold levels in order to even better suit patient needs.

WO-A-00/62708 discusses a multi-section stent. The stent includes a connecting structure to allow sections to move and flex relative to each other. On deployment the connecting structure may separate the stent sections by breaking or degrading within the body lumen.

In view of the above, a need exists for a stent having adjacent segments that remain connected during delivery until after deployment is effected so as to provide a more stable emplacement of the stent within a vessel or other body passageway. A need further exists to provide a stent having a frangible bridge connecting adjacent segments comprising the stent, wherein the bridge is comprised of metallic materials and dimensioned to accommodate intended strain threshold levels.

According to the present invention, there is provided an intraluminal medical device as defined in appended claim 1.

Various aspects of the invention comprise an intraluminal medical device having axially adjacent segments connected by at least one strain concentrating bridge, wherein the axially adjacent segments remain connected during delivery of the device to an intended treatment site. The at least one strain concentrating bridge yields to separate the axially adjacent segments when the device is placed in an area subjected to sufficient dynamic loading within the patient. Of course, if the device is placed in an area of minimal or low dynamic loading, then the device tends to remain intact. The medical device is a stent comprised of at least two axially adjacent segments.

The connected axially adjacent segments are connected by a bridge having a thinned portion. The bridge and thinned portion thereof are comprised of the same biocompatible materials as the axially adjacent segments but yield at the thinned portion of the bridge when subjected to sufficient dynamic loading. The bridge and thinned portion thereof is otherwise generally the same as that embodiment having the frangible bridge with a notched strain riser discussed above.

In some embodiments, the stent is comprised of self-expanding materials, such as a nickel-titanium alloy for example that sold under the trade name Nitinol, such that the stent is delivered to an intended treatment site in a constrained state, whereafter the stent recovers its expanded state within the vessel or other passageway in which the stent is emplaced. In other embodiments, the stent is comprised of plastically deformable materials, such that the stent is delivered to an intended treatment site in a constrained state that is maintained by bioabsorable restraints until the restraints are absorbed, whereafter the stent takes on its plastically expanded state. In still other embodiments, the stent is comprised of a balloon expandable stent that is otherwise generally the same as the self-expanding stent embodiment described herein. In any case, the number, shape and arrangement of the bridges may be altered, and portions of the bridges may include radiopaque materials or drug eluting, or other bio-active agent, in order to accommodate various medical and physiological needs.

The independent and unconnected nature of a discontinuous stent structure allows the shape of a stented segment to more closely approximate the shape of an unstented segment of a blood vessel or other passageway. A conventionally continuous stent structure does not easily accommodate abrupt localized changes in loading or deformation within its length because its bridging elements propagate these local effects to adjacent structures. A stent comprised of discontinuous segments, based on localized loading conditions, thus allows local effects to remain local upon yielding of bridging elements rather than axially transferring loads or deformations between rings of axially adjacent segments. This behavior more readily conforms each segment to the naturally occurring states of deflection of the vessel, or other passageway, in which the stent is emplaced. Healing and durability of clinical outcomes tends to be improved as a result.

The above and other features of the invention, including various novel details of construction and combinations of parts, will now be more particularly described with reference to the accompanying drawings and claims. It will be understood that the various exemplary embodiments of the invention described herein are shown by way of illustration only and not as a limitation thereof. The principles and features of this invention may be employed in various alternative embodiments without departing from the scope of the invention as claimed.

Embodiments of the invention will now be described by way of example, with reference to the accompanying drawings, in which:
Figure 1A illustrates a schematic view of one example of a U-shaped frangible bridge given as background to the invention.
Figure 1B illustrates aspects of the frangible bridge in a locally fractured state and in a connected state between adjacent segments of a stent.
Figure 1C illustrates a flat projection of a series of adjacent segments of a stent connected by the generally U-shaped frangible bridges of Fig. 1A.
Figure 2A illustrates a frangible bridge given as background to the present invention.
Figure 2B illustrates aspects of the frangible bridge of Fig. 2A connecting adjacent segments of a stent.
Figure 2C illustrates the separation of adjacent segments of the stent upon absorprtion or fracture of the frangible bridge of Fig. 2A.
Figure 3A illustrates a stent having a bridge with thinned portion connecting axially adjacent segments according to the invention.
Figure 3B is an inset of Fig. 3A illustrating in greater detail a bridge with thinned portion of Fig. 3A according to the invention.

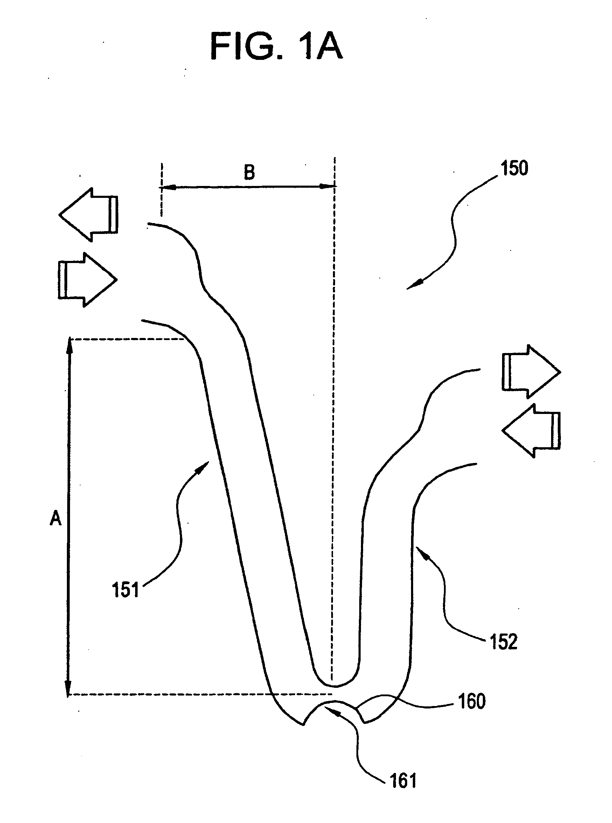

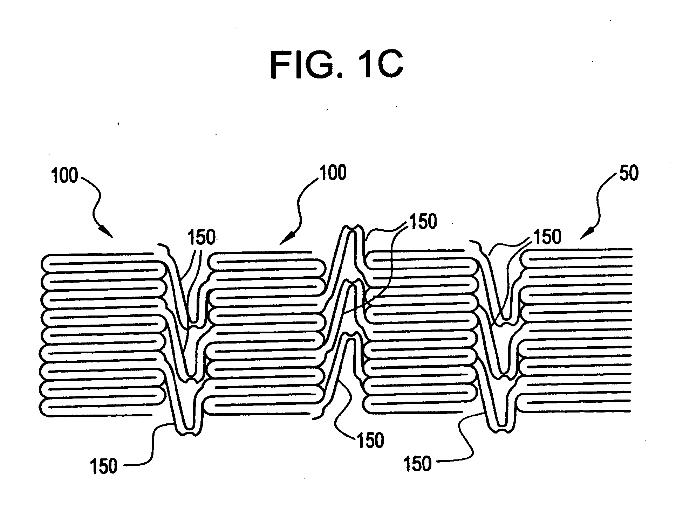

Figs. 1A-1C illustrate a stent 50 comprised of a series of axially adjacent segments 100. These segments 100 may be comprised of stainless steel or Nitinol, as in the Palmaz™ or Palmaz-Schatz^{™} stent made by Cordis Corporation or the Smart Stent ™, also made by Cordis Corporation. These segments 100 are intended to be of strong radial strength when emplaced within the body. The segments 100 may be self-expanding, may be plastically expanded by removal of a restraint, or may be expanded using a balloon catheter (not shown). In any event, the expansion of the segments 100 ideally occurs when the stent is positioned, as intended, within the vessel or other passageway of the patient.

In the example shown in Figs. 1A-1C, some of the axially adjacent segments 100 are connected by at least one frangible strain concentrating bridge 150. As shown in Figs. 1A-1C, three frangible bridges 150 are spaced around each segment 100. The artisan will appreciate, however, that other arrangements per segment 100 may be provided as where more or less than three frangible bridges 150 are provided per segment 100, so long as at least one bridge 150 is provided between adjacent segments 100. In this manner, some adjacent segments may be connected by more bridges 150 than other adjacent segments 100.

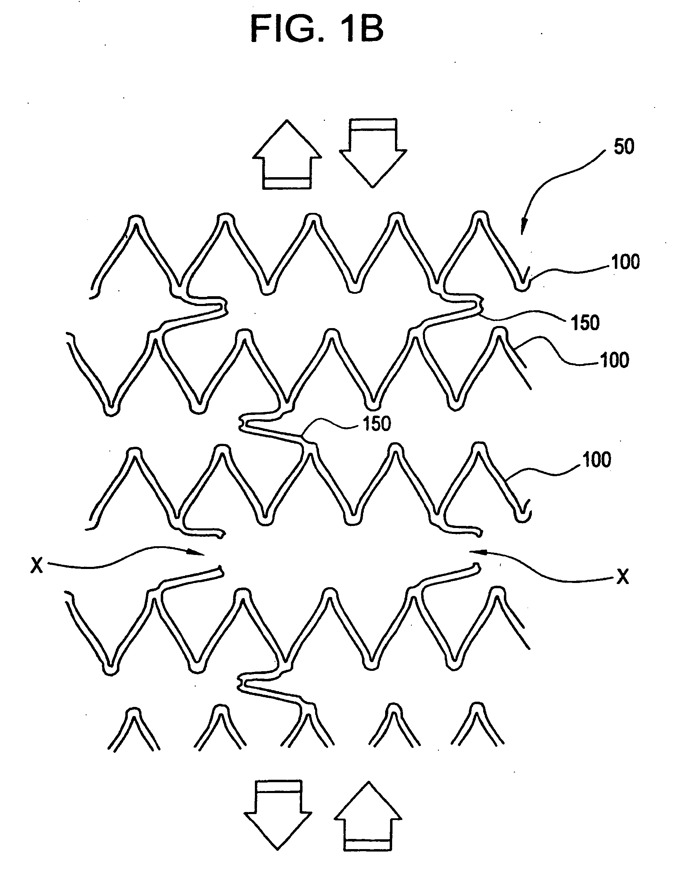

Each frangible bridge 150 is further comprised of a first bridge leg 151, a second bridge leg 152, and a notched strain riser 160 connecting the first and second bridge legs. The notched strain riser 160 is generally at an apex of the bridge 150 so as to connect the first and second bridge legs 151, 152 of the bridge 150. A position of weakness 161 is located within the arc of the strain riser 160. The strain riser 160 is provided with a predetermined threshold level of strain that the strain riser 160 can endure before yielding, i.e., fracturing, at its position of weakness 161. Such yielding causes the separation of adjacent segments 100 within the localized region of threshold exceeding strain. The position of weakness 161 can thus result in yielding, or fracture, of the strain riser 160 at any point along the arc thereof. Fig. 1B shows, for example, the fractured state X of certain bridges exposed to localized strain beyond a bridge's threshold level even as other bridges 150 remain in tact as other bridge threshold levels are not exceeded.

The predetermined threshold level of strain at position of weakness 161 is based upon several factors including the materials used to comprise the bridge 150, the shape of the notched strain riser 160, and the dimensions of the first and second bridge legs 151, 152. For example, lengthening one or both of the first bridge leg 151 and the second bridge leg 152, such as lengthening span A of the first bridge leg 151, tends to maximize the fulcrum, or moment, applied to the strain riser 160 such that the the predetermined threshold level of the strain riser 160 is reached sooner. As a result, the strains or stresses other portions of the stent 50 are subjected to tend to be reduced, or at least better distributed about the various axially adjacent segments 100 of the stent 50. Alternatively, lengthening the span (B) of the bridge 150 from one segment 100 to an approximately midway point of the strain riser 160 (Fig. 1A) can also alter the threshold level of the strain riser 160 such that increasing the span (B) tends to decrease the threshold level of the strain riser 160. Of course, the arc of the strain riser 160 can also be increased or decreased to alter the threshold level of the bridge 150, whereby increasing the arc tends to decrease the threshold level resulting in sooner yielding of the bridge 150. The artisan will appreciate that such strains can be localized such that only some of the bridges 150 yield, while others of the bridges 150 remain in tact.

The bridge 150, including the strain riser 160 and the first and second bridge legs 151, 152 is preferably comprised of one or more biocompatible metallic materials according to the invention. The biocompatible metals may be, for example, titanium, vanadium, aluminum, nickel, tantalum, zirconium, chromium, silver, gold, silicon, magnesium, niobium, scandium, platinum, cobalt, palladium, manganese, molybdenum, and alloys, or combinations thereof, or any other known or later developed biocompatible material suitable for use within the anatomy of a patient. The biocompatible material is most preferably bioabsorbable upon yielding so as not to undesirably impact the lumen of the vessel or other passageway in which the stent is emplaced. Radiopaque materials may be added to, or coated on, the bridge 150 or segments 100 of the stent 50 in order to accommodate visualization of the stent 50, or the bridge 150 in particular, as the stent 50 is emplaced within the vasculature or other passageway of a patient. Drugs, or other bio-active agents, may also be added to, or coated on, all or some of the bridges 150 or segments 100 of the stent 50 in order to even better meet medical or physiological needs.

When stents are emplaced within the vasculature or other passageway of a patient, cyclic strains occur due to the physiologic dynamics experienced by a patient. Longitudinal motions of the lumen causes the segments 100 of a stent 50 to expand and contract in the longitudinal direction, as indicated by the arrows of Figs. 1A & 1B, for example. The notched strain riser 160 thus acts as a focal point for the cyclic strain imposed during such loading conditions, as when the first bridge leg 151 or the second bridge leg 152 are deflected due to longitudinal motions of the vessel or other passageway in which the stent is emplaced. The notched strain riser 160 is designed to yield, or fracture, if the loading conditions exceed the predetermined threshold level of strain that the bridge 150 was designed to endure. In this manner, cyclic strains or other stresses are not propagated to the adjacent segments to which the frangible bridge was connected. Rather, the yielding of one or more of the frangible bridges 150 whose threshold level was exceeded enables the axially adjacent segments 100 to separate from those bridges, which minimizes potentially harmful fatigue fractures in the other segments 100 of the stent.

The stent 50 with at least one frangible bridge 150 connecting axially adjacent segments 100 as described herein is preferably made using conventional stent manufacturing methods. However, the notched strain riser 160 may be laser cut or etched into the frangible bridge 150 so that during emplacement within the vessel or other passageway of a patient the frangible bridge 150 is able to yield as intended. The stent 50, including any portion thereof, can be loaded with drugs or other bioactive agents as is well-appreciated in the art.

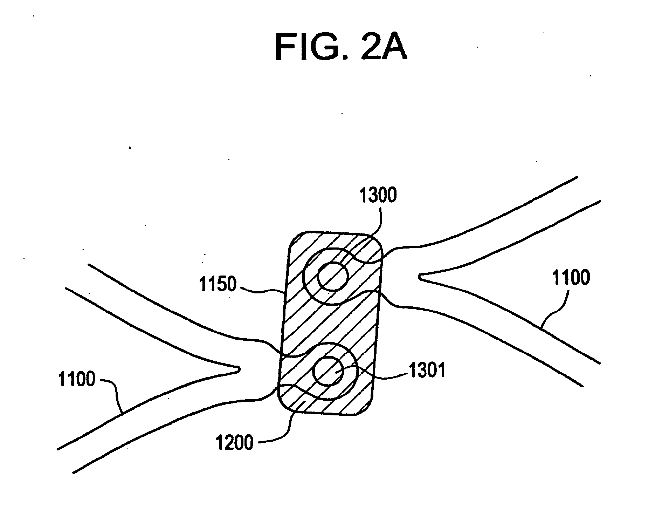

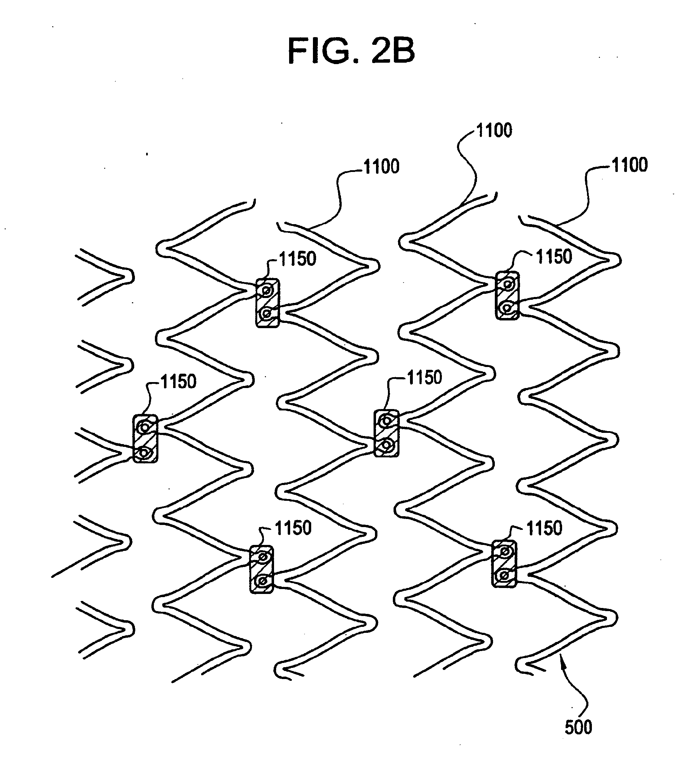

Figs. 2A-2C are included as background to the invention and illustrate a stent with discontinous segments connected by at least one strain concentrating bridge. The stent 500 is generally the same as stent 50 described above except that the at least one bridge 1150 is comprised of a slotted member 1200 into which protrusions 1300, from axially adjacent segments 1100, are fitted. As before, the at least one bridge 1150 is comprised of one or more known or later developed biocompatible metallic materials, alloys, or combinations thereof, that are most preferably bioabsorbable upon yielding so as not to undesirably impact the lumen of the vessel, or other passageway, in which the stent 500 is emplaced. Radiopaque materials, drugs or other bio-active agents may be added to, or coated on, some or all of the at least one bridge or segments of the stent to enhance visualization thereof, or to even better meet medical or physiological needs. The protrusions 1300 may include a hole 1301, for example, in which such radiopaque materials, drugs or other agents, may be received.

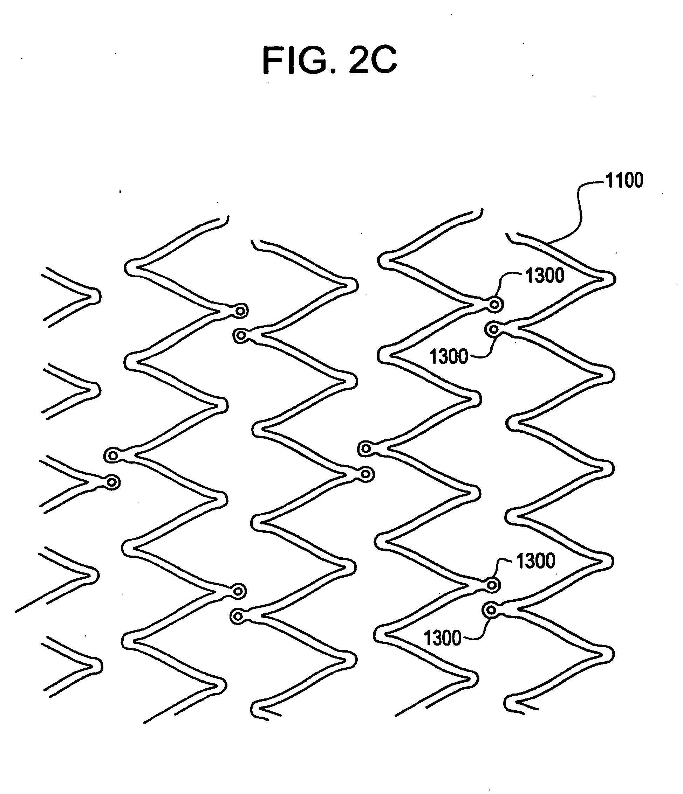

During manufacture, the various adjacent segments 1100 are positioned juxtaposed one to the other as in Fig. 2C, for example. The slotted members 1200 of the bridges 1150 are then fused directly to the intended adjacent segments 1100 so as to surround the protrusions 1300 and, where applicable, fill the holes 1301 with radiopaque materials, drugs or other agents. In this manner, the stent is continuous during delivery but may become locally discontinuous upon yielding of any of the at least one bridge 1150 when subjected to sufficient strain. The biocompatible metallic materials comprising the bridge 1150 has a lower threshold of cyclic strain than does the materials comprising the segments 1100 or the protrusions 1300 so as to reduce the likelihood that cyclic strains, or other loading conditions, are not undesirably transferred to adjacent segments.

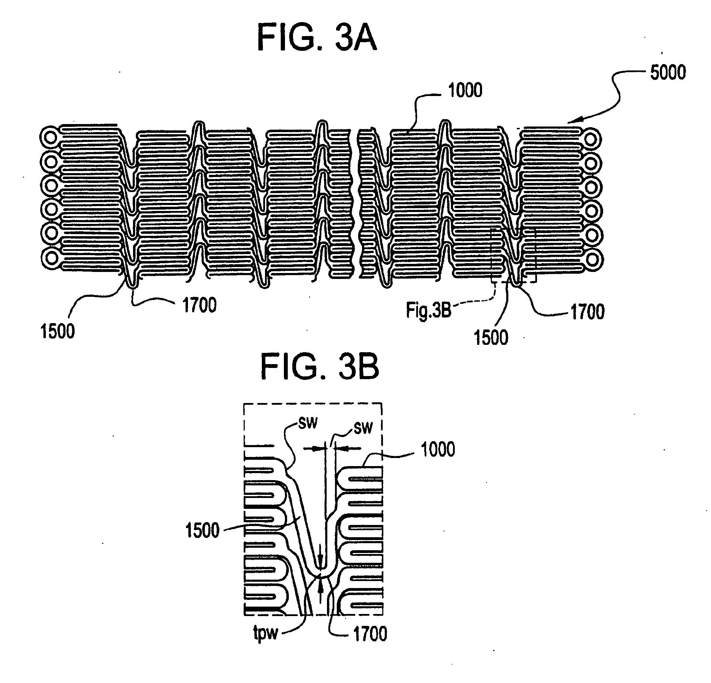

Figs. 3A and 3B illustrate an embodiment of a stent according to the invention with segments connected by at least one strain concentrating bridge. The embodiment shown in Figs. 3A and 3B is generally the same as that shown and described above with respect to Figs. 1A-1C, except that in Figs. 3A and 3B the bridge 1500 includes a thinned portion 1700 rather than the notched strain riser 160 of Figs. 1A-1C.

As shown in Fig. 3A, the stent 5000 is comprised of axially adjacent segments 1000, at least some of which are connected by a generally U-shaped bridge 1500. A portion of the U-shaped bridge 1500 includes the thinned portion 1700 of the bridge. Although shown in Figs. 3A and 3B as having the thinned portion 1700 at an apex of the U-shaped bridge 1500, the thinned portion 1700 could be other than at the apex of the bridge 1500. The bridge 1500 and the thinned portion 1700 thereof, are preferably comprised of the same biocompatible materials as comprise the axially adjacent segments 1000. The thinned portion 1700 of the bridge 1500 is uniformly thinned to a thinned portion width (tpw) less than the segment width (sw) of the axially adjacent segments 1000.
Fig. 3B is an inset of the boxed area of Fig. 3A, wherein the Fig. 3B inset shows in greater detail the relationship of the segments 1000, the bridge 1500 and the thinned portion 1700 thereof. As shown in Fig. 3B, the segment width (sw) of the segments 1000 is approximately 0.137 mm (0.0054 in.), for example, which sw continues through the bridge 1500 until reaching the thinned portion width (tpw) of approximately 0.104 mm (0.0041 in.), for example. Of course other segment widths (sw) and thinned portion widths (tpw) are available to provided load bearing capacity, or yield tendencies, of the bridge 1500 and the thinned portion thereof, according to medical and physiological needs, as the artisan should readily appreciate. As before, the bridge 1500, the thinned portion 1700 thereof, or the segments 1000 of the stent 5000 may have radiopaque materials, drugs or other agents incorporated therein or coated thereon, to increase the visualization and thereapeutic effect of the stent.

After delivery to an intended treatment site, the stent 50, 500 or 5000 is expanded using conventional methods such as balloon catheters, self-expanding materials, or plastically expanded materials after degradation of a restraint. In either event, after the stent is expanded in the lumen of a vessel, or other passageway, the bridge is subjected to naturally occurring corrosive forces in the body. Together with the physiologic dynamics, these corrosive forces tend to breakdown the metallic materials of the bridge after a period of time. Ideally, when subjected to sufficient loads after such time, one or more of the bridges yield permitting certain of the adjacent segments to separate. The separation of adjacent segments in this manner enables the stent to more readily accommodate the physiologic dynamics of the vessel, or other passageway, in which the stent is emplaced.

Because the strain concentrating bridge of the various embodiments described herein acts as a flexible hinge, it may also improve deployment characteristics of the stent. This bridges described herein may be somewhat more flexible during delivery than a standard connector member, so the stent may be able to negotiate through more difficult lumens as compared to prior stents. The bridges described herein thus concentrates strain in the bridge more quickly and with a greater magnitude than at other areas of the stent during bending, tension, compression, or torsion of the stent. Because peak strains are experienced at the bridge, the bridge yields to preserve the integrity of the other structures of the stent, such as the radially load bearing axially adjacent segments. As constructed, the combined structure of the stent will act as a single stent during delivery and deployment. However, after the at least one strain concentrating bridge is absorbed, the axially adjacent segments become unconnected, discontinuous and independent of one another. This may be advantageous in vessels subject to longitudinal elongation compressing or bending.

Furthermore, when combined with drug eluting technology, the at least one bridge may provide an additional drug delivery reservoir for the stent. A bolus of drug may be contained on or in some or all of the bridges for delivery to the body upon absorption of the bridge into the body.

## Claims

1. A stent (5000) comprising:
a series of at least two axially adjacent segments (1000);
at least one U-shaped strain concentrating bridge (1500) connecting the at least two axially adjacent segments; and
a thinned portion (1700) of the at least one bridge that yields when subjected to localized loads beyond a load bearing capacity of the thinned portion, **characterised in that**:
the at least two axially adjacent segments (1000), the at least one bridge (1500) and the respective thinner portion (1700) thereof, are comprised of biocompatible metal materials consisting of at least one of titanium, vanadium, aluminum, nickel, tantalum, zirconium, chromium, silver, gold, silicon, magnesium, niobium, scandium, platinum, cobalt, palladium, manganese, molybdenum, and alloys thereof;
**in that** the thinned portion (1700) of a respective at least one bridge (1500) is comprised of a thinned portion width thinner (tpw) than a segment width (sw) of the at least two axially adjacent segments (1000);
and **in that** the thinned portion (1700) of a respective at least one bridge (1500) is comprised of a thinned portion width (tpw) uniformly thinner than a segment width (sw) of the at least two axially adjacent segments (1000).

2. The stent (5000) of claim 1, wherein the thinned portion (1700) is at an apex of the U-shape of the at least one bridge (1500).

3. The stent (5000) of claim 1, further comprising radiopaque materials, drugs or other agents incorporated into or onto at least one of the at least two axially adjacent segments (1000), the at least one bridge (1500) and the respective thinned portion (1700) thereof.

## Patentansprüche

1. Stent (5000), der Folgendes umfasst:
eine Folge von zumindest zwei axial benachbarten Segmenten (1000);
zumindest eine U-förmige belastungskonzentrierende Brücke (1500), welche die zumindest zwei axial benachbarten Segmente verbindet; und
einen verdünnten Teilbereich (1700) der zumindest einen Brücke, welcher nachgibt, wenn er lokalisierten Lasten jenseits einer Lasttragekapazität des verdünnten Teilbereichs ausgesetzt ist, **dadurch gekennzeichnet, dass**:
die zumindest zwei axial benachbarten Segmente (1000), die zumindest eine Brücke (1500) und der jeweilige dünnere Teilbereich (1700) davon biokompatible metallische Materialien umfassen, welche aus Titan, Vanadium, Aluminium, Nickel, Tantal, Zirkonium, Chrom, Silber, Gold, Silizium, Magnesium, Niob, Skandium, Platin, Kobalt, Palladium, Mangan, Molybden und/oder Legierungen davon bestehen;
dass der verdünnte Teilbereich (1700) einer jeweiligen zumindest einen Brücke (1500) eine Breite (tpw) des verdünnten Teilbereichs umfasst, welche dünner ist als eine Segmentbreite (sw) der zumindest zwei axial benachbarten Segmente (1000);
und dass der verdünnte Teilbereich (1700) einer jeweiligen zumindest einen Brücke (1500) eine Breite (tpw) des verdünnten Teilbereichs umfasst, welche gleichmäßig dünner ist als eine Segmentbreite (sw) der zumindest zwei axial benachbarten Segmente (1000).

2. Stent (5000) nach Anspruch 1, wobei der verdünnte Teilbereich (1700) sich an einem Scheitel der U-Form der zumindest einen Brücke (1500) befindet.

3. Stent (5000) nach Anspruch 1, welcher weiter röntgenstrahlenundurchlässige Materialien, Medikamente oder andere Wirkstoffe umfasst, welche in oder auf zumindest einem der zumindest zwei axial benachbarten Segemente (1000), der zumindest einen Brücke (1500) und/oder dem jeweiligen verdünnten Teilbereich (1700) davon untergebracht sind.

## Revendications

1. Stent (5000) comprenant :
une série d'au moins deux segments axialement adjacents (1000) ;
au moins un pont de concentration de contrainte en forme de U (1500) raccordant les au moins deux segments axialement adjacents ; et
une partie amincie (1700) du au moins un pont qui est élastique lorsqu'elle est soumise à des charges localisées au-delà d'une capacité de support de charge de la partie amincie, **caractérisé en ce que** :
les au moins deux segments axialement adjacents (1000), le au moins un pont (1500) et sa partie amincie (1700) respective sont composés de matériaux métalliques biocompatibles comprenant au moins l'un parmi le titane, le vanadium, l'aluminium, le nickel, le tantale, le zirconium, le chrome, l'argent, l'or, le silicium, le magnésium, le niobium, le scandium, le platine, le cobalt, le palladium, le manganèse, le molybdène et leurs alliages ;
**en ce que** la partie amincie (1700) du au moins un pont (1500) respectif est composée d'une largeur de partie amincie plus fine (tpw) qu'une largeur de segment (sw) des au moins deux segments axialement adjacents (1000) ;
et **en ce que** la partie amincie (1700) du au moins un pont (1500) respectif est composée d'une largeur de partie amincie (tpw) uniformément plus fine qu'une largeur de segment (sw) des au moins deux segments axialement adjacents (1000).

2. Stent (5000) selon la revendication 1, dans lequel la partie amincie (1700) est située au niveau d'un sommet de la forme de U du au moins un pont (1500).

3. Stent (5000) selon la revendication 1, comprenant en outre des matériaux radio-opaques, des médicaments ou d'autres agents incorporés dans ou sur au moins l'un parmi les au moins deux segments axialement adjacents (1000), le au moins un pont (1500) et sa partie amincie (1700) respective.
